(19) **Europäisches Patentamt / European Patent Office / Office européen des brevets**

(11) **EP 4 751 743 A1**

(12) **EUROPEAN PATENT APPLICATION**
published in accordance with Art. 153(4) EPC

(43) Date of publication:
03.06.2026 Bulletin 2026/23

(21) Application number: 24845628.7

(22) Date of filing: 23.07.2024

(51) International Patent Classification (IPC):
$A61L\ 27/42^{(2006.01)}$    $A61L\ 27/04^{(2006.01)}$
$A61L\ 27/30^{(2006.01)}$    $A61L\ 27/54^{(2006.01)}$
$A61L\ 27/58^{(2006.01)}$

(52) Cooperative Patent Classification (CPC):
A61L 27/04; A61L 27/30; A61L 27/42; A61L 27/54;
A61L 27/58

(86) International application number:
PCT/JP2024/026354

(87) International publication number:
WO 2025/023253 (30.01.2025 Gazette 2025/05)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 26.07.2023 JP 2023121668

(71) Applicant: Denka Company Limited
Tokyo 103-8338 (JP)

(72) Inventors:
• MIYATA, Kenji
Tokyo 103-8338 (JP)
• ITO, Kazuhiro
Tokyo 103-8338 (JP)

(74) Representative: Hoffmann Eitle
Patent- und Rechtsanwälte PartmbB
Arabellastraße 30
81925 München (DE)

(54) **METHOD FOR PRODUCING BIOCOMPATIBLE MATERIAL, AND BIOCOMPATIBLE MATERIAL**

(57) [Problem] To provide a biocompatible material having sufficient osteoconductivity and antibacterial properties, and a method for producing the biocompatible material.

[Solution] Provided according to one embodiment of the present invention is a method for producing a biocompatible material, the method comprising: a step for preparing a base and particles that contain, as a main component, a compound containing silicon and nitrogen; and a step for causing the particles to collide with the surface of the base by aerosol deposition and forming a coating layer that covers at least part of the base, thus obtaining a biocompatible material.

FIG. 1

## Description

[Technical Field]

The present disclosure relates to a method for producing a biocompatible material, and to a biocompatible material.

[Background Art]

Conventionally, sintered bodies of hydroxyapatite have been used as biocompatible materials (see Patent Literature 1). Hydroxyapatite has excellent biocompatibility and high osteoconductivity. However, hydroxyapatite does not exhibit antibacterial properties; therefore, after transplantation of a biocompatible material into a living body, administration of an antibacterial agent or the like is carried out to prevent onset of an infectious disease. On the other hand, biocompatible materials made of titanium or a titanium alloy also exist, but cell proliferation capability and osteoconductivity thereof are lower compared with biocompatible materials made of hydroxyapatite.

## PRIOR ART DOCUMENTS

Patent Literature

[Patent Literature 1] Patent Literature 1: JP 2003-286073 A

[Problems to be Solved by the Invention]

In view of the circumstances above, the present disclosure provides a biocompatible material having sufficient osteoconductivity and antibacterial properties and a method for producing the same.

[Solution to Problem]

According to one aspect of the present disclosure, there is provided a method for producing a biocompatible material comprising: preparing a base and particles containing, as a main component, a compound including silicon and nitrogen; and forming, by causing the particles to collide with a surface of the base by an aerosol deposition method so as to adhere thereto, a coating layer that covers at least a part of the base, thereby obtaining the biocompatible material.
According to this aspect, a biocompatible material having sufficient osteoconductivity and antibacterial properties can be obtained.

## BRIEF DESCRIPTION OF DRAWINGS

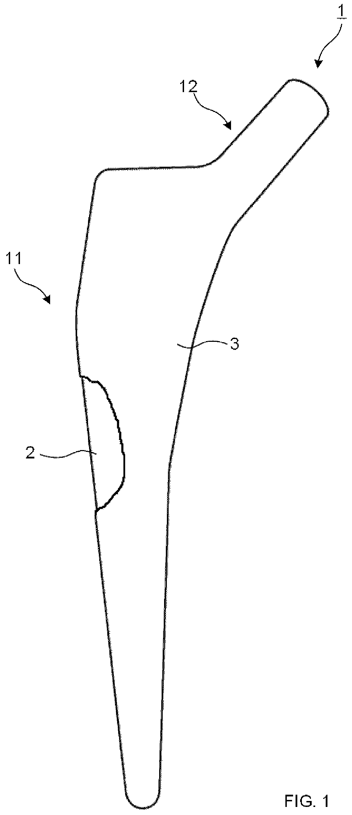

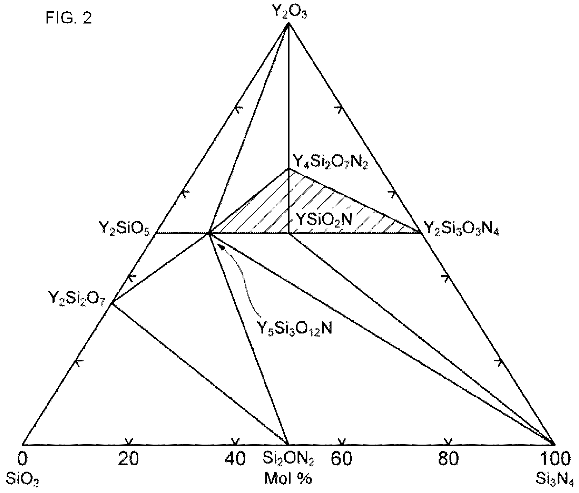

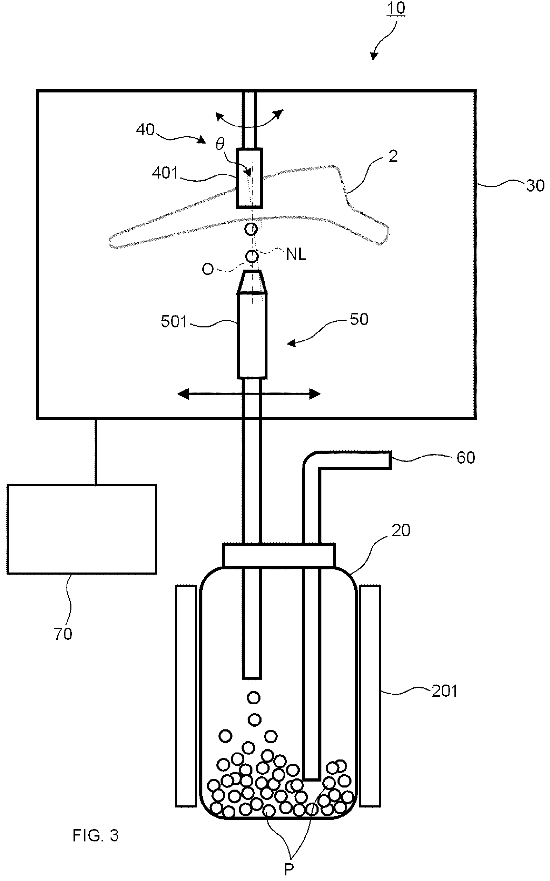

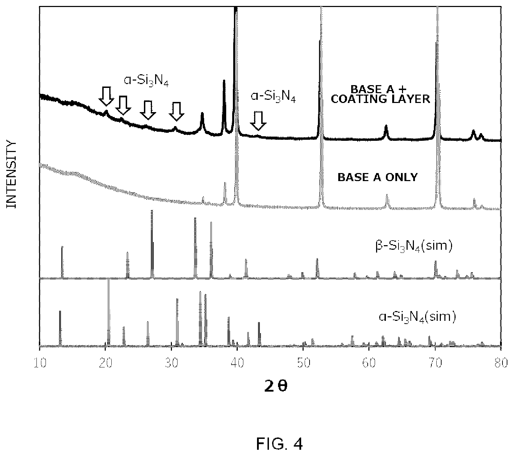

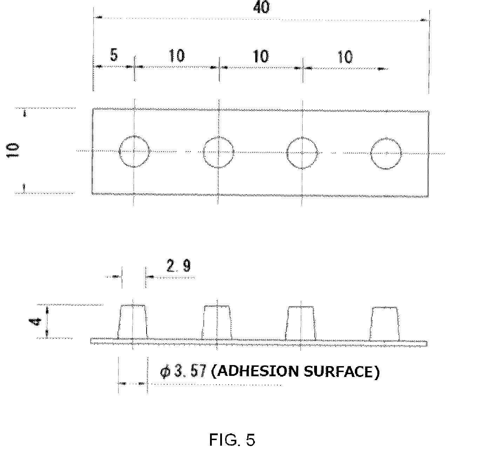

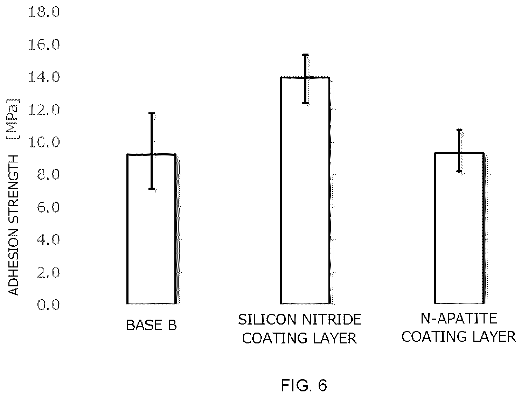

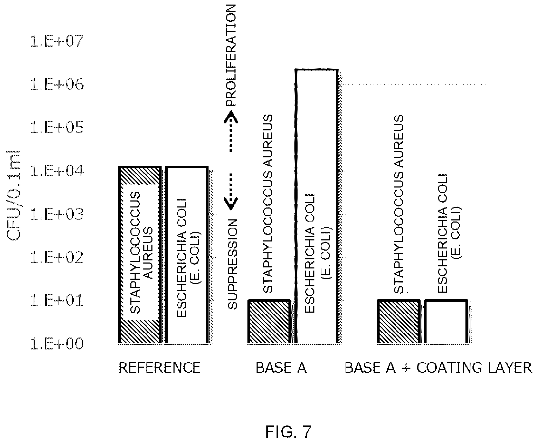

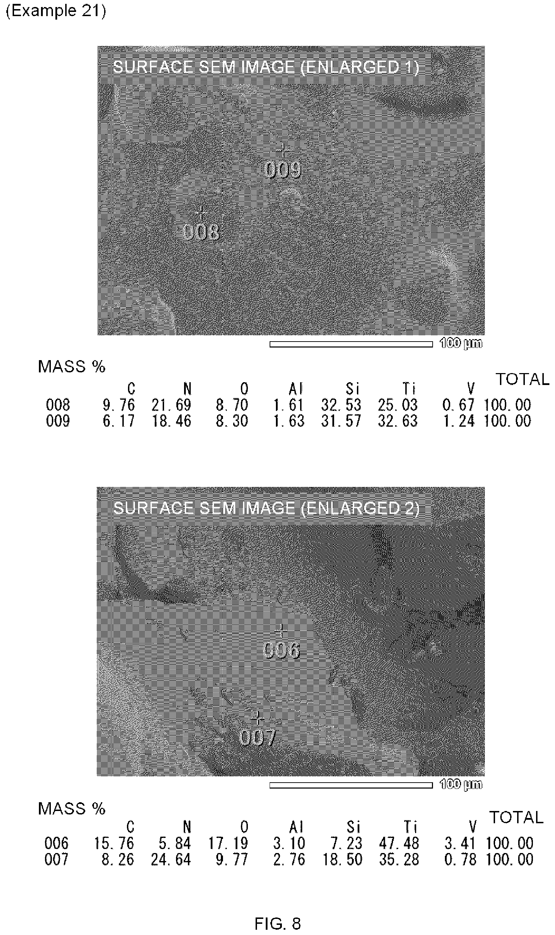

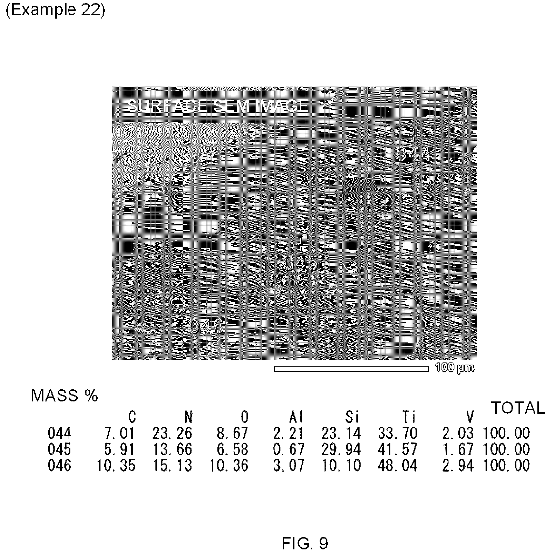

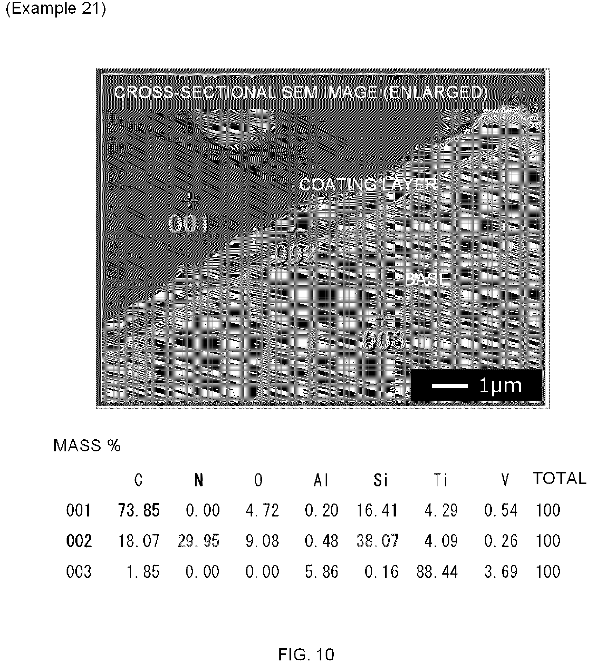

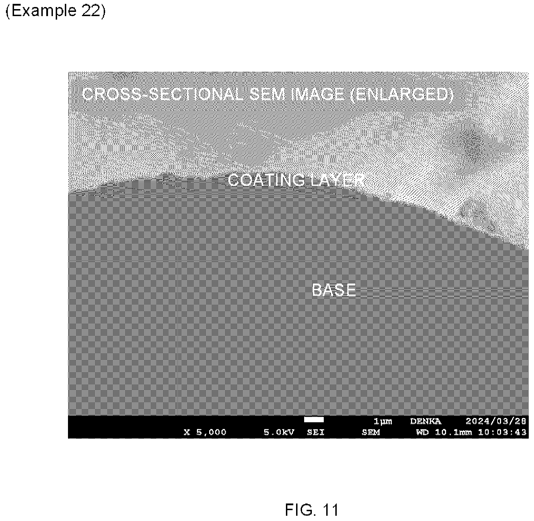

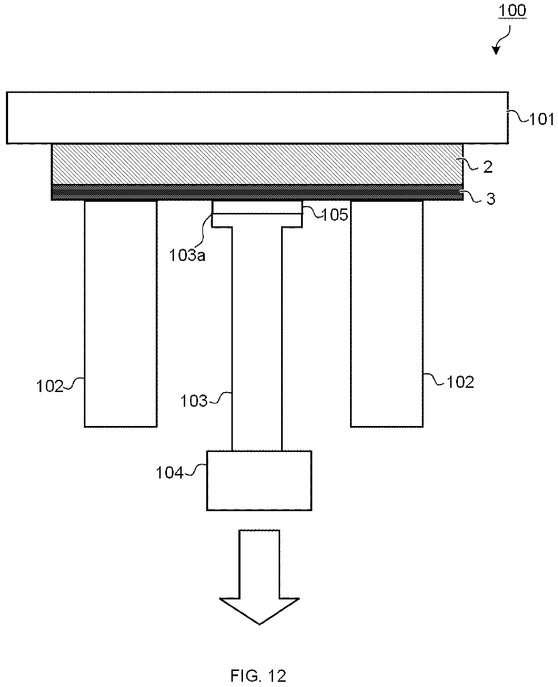

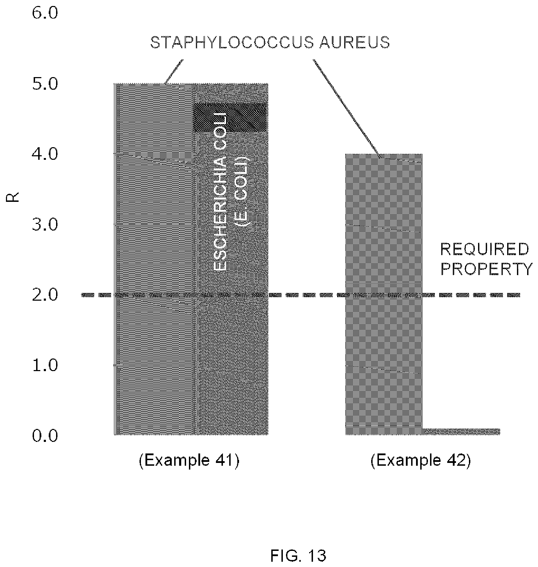

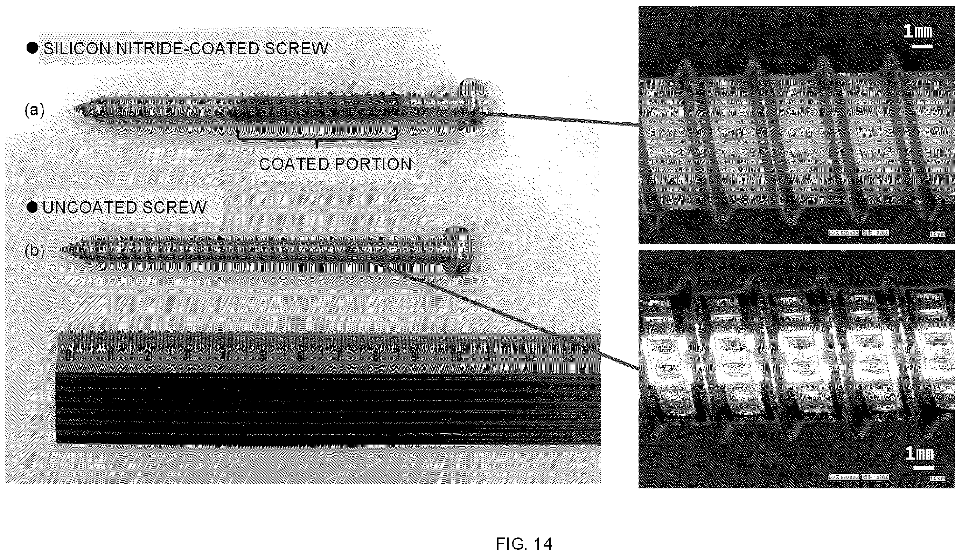

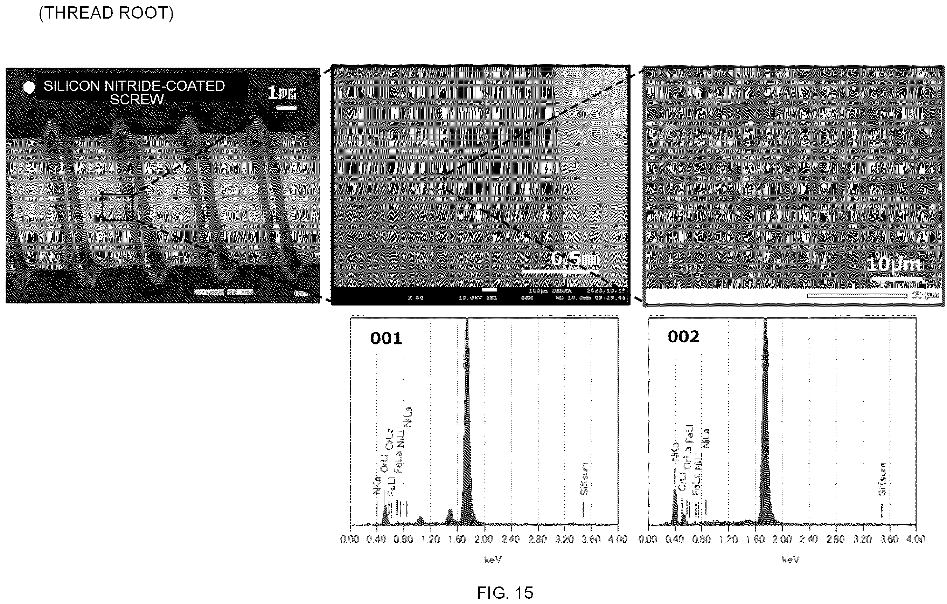

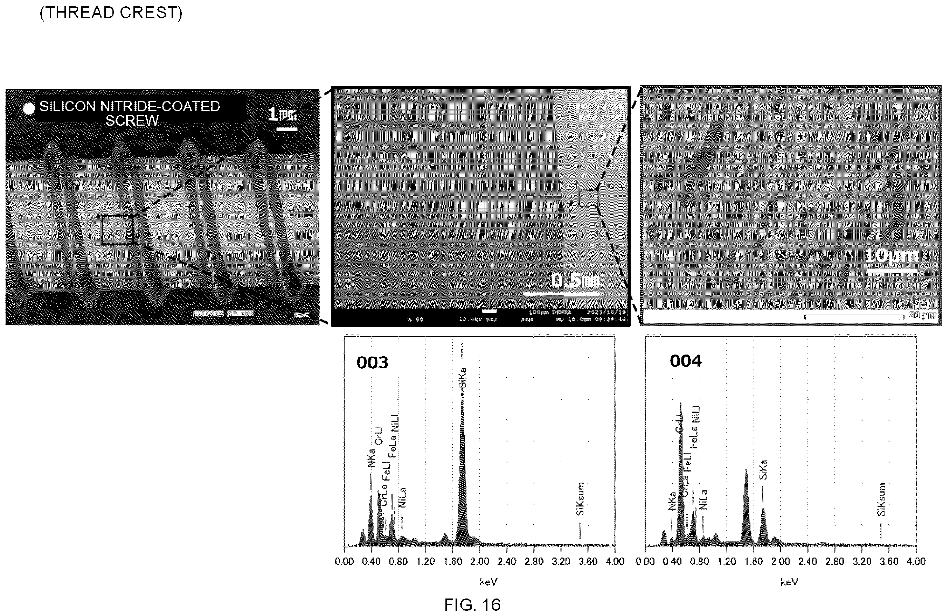

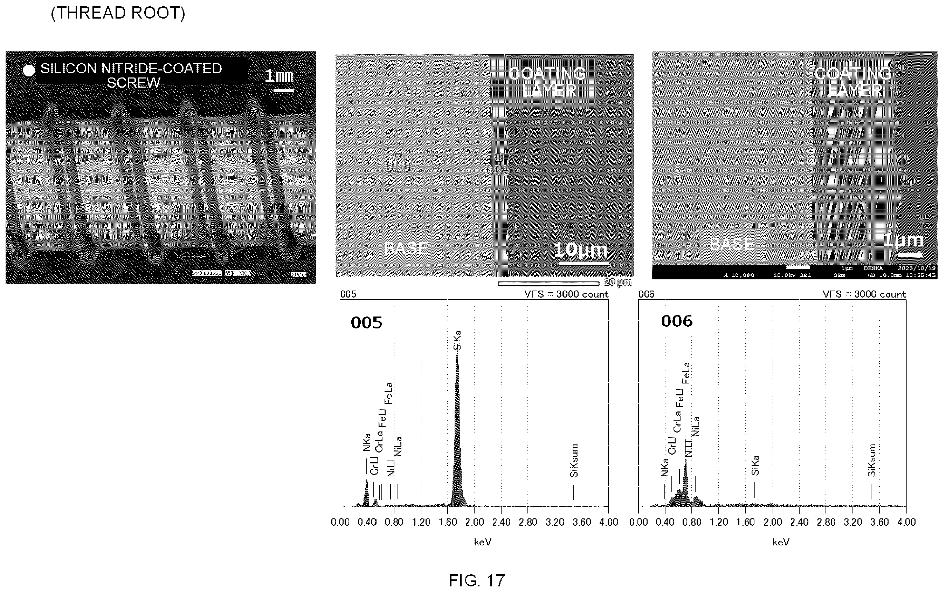

FIG. 1 is a front view (partially cut away) showing an embodiment of a biocompatible material. FIG. 2 is a phase diagram of a ternary system of $Si_3N_4$, $SiO_2$, and $Y_2O_3$. FIG. 3 is a schematic diagram showing an example of an aerosol deposition apparatus. FIG. 4 is a graph showing results of XRD measurement for samples. FIG. 5 is a schematic diagram showing a method of an adhesion test for samples. FIG. 6 is a graph showing results of adhesion strength measurements for samples. FIG. 7 is a graph showing results of antibacterial tests for samples. FIG. 8 is a view showing results of surface SEM observation and composition analysis for a sample of Example 21. FIG. 9 is a view showing results of surface SEM observation and composition analysis for a sample of Example 22. FIG. 10 is a view showing results of cross-sectional SEM observation and composition analysis for a sample of Example 21. FIG. 11 is a view showing results of cross-sectional SEM observation and composition analysis for a sample of Example 22. FIG. 12 is a schematic diagram showing a configuration of an adhesion strength test apparatus. FIG. 13 is a graph showing results of antibacterial tests for samples of Examples 41 and 42. FIG. 14 is a photograph showing external appearances of a screw before and after formation of a coating layer. FIG. 15 is a view showing results of surface SEM observation and composition analysis at the thread root of the coated screw. FIG. 16 is a view showing results of surface SEM observation and composition analysis at the thread crest of the coated screw. FIG. 17 is a view showing results of cross-sectional SEM observation and composition analysis at the thread root of the coated screw.

## DETAILED DESCRIPTION

Hereinafter, embodiments of the present disclosure are described with reference to the drawings. Various features described in the embodiments below can be combined with one another.

First, a biocompatible material (implant) manufactured by the production method of the present embodiment is described.

FIG. 1 is a front view (partially cut away) showing an embodiment of a biocompatible material. FIG. 2 is a phase diagram of a ternary system of $Si_3N_4$, $SiO_2$, and $Y_2O_3$.

A biocompatible material 1 shown in FIG. 1 is a component (stem) of an artificial joint and has an insertion portion 11 to be inserted into a femur and a mounting portion 12 to which an artificial femoral head is attached.

The biocompatible material 1 includes a base 2 and a coating layer 3 that covers at least a part (in this embodiment, the entirety) of the base 2. Then, the coating layer 3 is formed using particles containing, as a main component, a compound including silicon and nitrogen. Accordingly, the coating layer 3 also contains, as a main component, a compound including silicon and nitrogen.

As a constituent material of the base 2, a metallic material or a resin material that has no or very low biotoxicity is suitably employed.

Examples of metallic materials include, for example, titanium, cobalt, chromium, tantalum, iron, nickel, copper, palladium, and platinum, which may be used singly or in combination of two or more (as an alloy).

On the other hand, examples of resin materials include, for example, polyetheretherketone (PEEK), polymethyl methacrylate (PMMA), polyarylamide (PARA), polycarbonate (PC), polyethylene (PE), and polylactic acid (PLA), which may be used singly or in combination of two or more.

According to investigations by the inventors, a compound including silicon and nitrogen has been found to have sufficient osteoconductivity and antibacterial properties. Therefore, when a biocompatible material 1 has a surface constituted by a coating layer 3 containing such a compound as a main component, fusion with bone at an application site (for example, a transplantation site) can be caused at an early stage while favorably preventing onset of an infectious disease accompanied by transplantation of the biocompatible material 1.

Such a compound preferably further contains yttrium and oxygen, namely is a composite compound (composite oxide) containing yttrium, oxygen, silicon, and nitrogen as constituent elements. By using such a composite compound, osteoconductivity and antibacterial properties of the biocompatible material 1 are further improved.

The composite compound may contain at least one selected from the group consisting of $Si_3N_4$-$SiO_2$-$Y_2O_3$-based compounds and $Si_3N_4$-$Y_2O_3$-based compounds. Here, the $Si_3N_4$-$SiO_2$-$Y_2O_3$-based compounds are composite compounds included in a phase diagram of the ternary system of $Si_3N_4$, $SiO_2$, and $Y_2O_3$ shown in FIG. 2.

The composite compound preferably has a composition within a region indicated by hatching in FIG. 2 (including a boundary line). The region is a triangular region defined by three points of $Y_5Si_3O_{12}N$ (N-apatite), $Y_2Si_3O_3N_4$ (N-melilite), and $Y_4Si_2O_7N_2$ (N-YAM). The triangular region includes compositions on the boundary line of the region.

The composite compound may contain at least one selected from the group consisting of $Y_5Si_3O_{12}N$ (N-apatite), $YSiO_2N$ (N-wollastonite), $Y_2Si_3O_3N_4$ (N-melilite), and $Y_4Si_2O_7N_2$ (N-YAM) on the boundary line of the triangular region. Thus, cell proliferation capability can be enhanced while cytotoxicity is sufficiently reduced. Further, differentiation of undifferentiated mesenchymal stem cells into osteoblasts, namely osteoconductivity (calcification), can be promoted. Therefore, such a composite compound is suitable as a constituent material of the coating layer 3 (particles).

In this specification, "main component" refers to a component contained in the largest amount when a plurality of components are contained. Components other than the "main component" are referred to as "subcomponents". The coating layer 3 (particles) may be constituted only of the composite compound serving as the main component, or may contain a subcomponent different from the main component.

A content of the composite compound in the coating layer 3 may be 90 mass% or more, 95 mass% or more, 98 mass% or more, 99 mass% or more, 99.5 mass% or more, or 99.7 mass% or more. The higher the content of the composite compound in the coating layer 3, the more likely biocompatibility is improved. Examples of subcomponents that may be contained in the coating layer 3 include silicon nitride, silicon dioxide, and yttrium oxide. A composite oxide in which two of these three components are composited may also be contained.

The compound may further contain silicon nitride.

A content of silicon nitride in the coating layer 3 may be 90 mass% or more, 95 mass% or more, 98 mass% or more, 99 mass% or more, 99.5 mass% or more, or 99.7 mass% or more. The higher the content of silicon nitride in the coating layer 3, the more likely antibacterial properties are improved. Examples of subcomponents that may be contained in the coating layer 3 include fluorine, chlorine, oxygen, iron, aluminum, and calcium. These may form a compound.

In this case, a content of fluorine in the coating layer 3 is preferably 900 mass ppm or less. The content of fluorine in the coating layer 3 may be 700 mass ppm or less, 500 mass ppm or less, 300 mass ppm or less, 250 mass ppm or less, 100 mass ppm or less, or 60 mass ppm or less. The lower the content of fluorine in the coating layer 3, the more likely antibacterial properties are improved. One of the factors is considered to be that, as the content of fluorine in the coating layer 3 decreases, when silicon nitride is hydrolyzed upon contact with moisture (for example, blood), a ratio of ammonia ($NH_3$) to ammonium ion ($NH_4^+$) becomes larger.

The content of fluorine in the coating layer 3 varies depending on a raw material composition used when producing the particles used to form the coating layer 3 and on presence or absence of surface treatment of the particles.

For example, by sufficiently washing the particles with a washing liquid such as water, the content of fluorine in the particles, and hence the content of fluorine in the coating layer 3, can be reduced.

The content of fluorine in the coating layer 3 may be 10 mass ppm or more, 20 mass ppm or more, or 30 mass ppm or more. Thus, the production cost of the coating layer 3 (biocompatible material 1) can be reduced.

A range of the content of fluorine in the coating layer 3 may be 10 mass ppm or more and 900 mass ppm or less.

A content of chlorine in the coating layer 3 may be 30 mass ppm or more, 50 mass ppm or more, 100 mass ppm or more, 300 mass ppm or more, 400 mass ppm or more, or 500 mass ppm or more. The higher the content of chlorine in the coating layer 3, the more likely antibacterial properties, particularly against Gram-negative bacteria, are improved.

The content of chlorine in the coating layer 3 varies depending on a raw material composition used when producing the particles used to form the coating layer 3 and on presence or absence of surface treatment of the particles.

The content of chlorine in the coating layer 3 may be 1000 mass ppm or less, 800 mass ppm or less, or 700 mass ppm or less. Thus, corrosion derived from chlorine can be suppressed. A range of the content of chlorine in the coating layer 3 may be 30 mass ppm or more and 1000 mass ppm or less.

A content of oxygen in the coating layer 3 may be 2 mass% or less, 1.5 mass% or less, or 1 mass% or less. Oxygen may be contained as, for example, silicon dioxide ($SiO_2$). The lower the content of oxygen in the coating layer 3, the more likely antibacterial performance is improved.

The content of oxygen in the coating layer 3 varies depending on a raw material composition used when producing the particles used to form the coating layer 3 and on a firing atmosphere. For example, when synthesizing particles (silicon nitride) by a direct nitriding method, if an oxygen concentration in the firing atmosphere is reduced, a content of oxygen in the particles (coating layer 3) tends to be reduced.

The content of oxygen in the coating layer 3 may be 0.2 mass% or more, 0.4 mass% or more, or 0.5 mass% or more. Thus, raw materials and gases having lower purities can be used, and the production cost of the particles and hence of the biocompatible material 1 can be reduced. A range of the content of oxygen in the coating layer 3 may be 0.2 mass% or more and 2 mass% or less.

A total content of aluminum, iron, and calcium in the coating layer 3 may be 2000 mass ppm or less or 1800 mass ppm or less. By reducing the total content of these, a content of silicon nitride in the coating layer 3 increases, and antibacterial properties tend to be improved.

The total content of aluminum, iron, and calcium in the coating layer 3 may be 10 mass ppm or more, 50 mass ppm or more, 500 mass ppm or more, or 1000 mass ppm or more. Thus, raw materials having lower purities can be used, and the production cost of the particles and hence of the biocompatible material 1 can be reduced.

A range of the total content of aluminum, iron, and calcium in the coating layer 3 may be 10 mass ppm or more and 2000 mass ppm or less.

Each content of fluorine, chlorine, oxygen, aluminum, iron, and calcium in the particles can be measured by the methods described in the Examples. The particles and the coating layer 3 may contain subcomponents other than those described above.

An α-phase ratio of silicon nitride (a relative ratio of α-$Si_3N_4$ to total $Si_3N_4$) may be 80% or more, 85% or more, 88% or more, or 90% or more. Increasing the α-phase ratio tends to improve antibacterial properties against Gram-negative bacteria.

The α-phase ratio of silicon nitride may be 99% or less, 97% or less, or 96% or less. Thus, the production cost of the particles and hence of the biocompatible material 1 can be reduced.

An example of the α-phase ratio of silicon nitride may be 80% or more and 99% or less. The α-phase ratio of silicon nitride can be adjusted, for example, by changing firing conditions (for example, firing temperature and firing time) when synthesizing silicon nitride (particles) by firing.

When an arithmetic average roughness Ra of the surface of the base 2 is defined as RaB [pm] and an arithmetic average roughness Ra of the surface of the coating layer 3 is defined as RaC [pm], RaC/RaB is preferably 0.9 or more and 1.5 or less, more preferably 0.92 or more and 1.3 or less, and still more preferably 0.94 or more and 1.1 or less. In this case, a value of RaC can be set to be large in accordance with a value of RaB. Therefore, undifferentiated mesenchymal stem cells more easily adhere to the coating layer 3 (biocompatible material 1), whereby differentiation and proliferation of the undifferentiated mesenchymal stem cells into osteoblasts can be promoted. Namely, osteoconductivity and cell proliferation capability of the biocompatible material 1 can be further improved. Such a coating layer 3 can be smoothly and reliably formed, for example, by an aerosol deposition method (AD method) described later.

Specifically, RaB of the surface of the base 2 is preferably 0.005 μm or more and 300 μm or less, more preferably 0.01 μm or more and 150 μm or less, and still more preferably 0.05 μm or more and 50 μm or less.

On the other hand, Ra of the surface of the coating layer 3 is preferably 0.01 μm or more and 300 μm or less, more preferably 0.05 μm or more and 150 μm or less, and still more preferably 0.1 μm or more and 50 μm or less. In this case, osteoconductivity and cell proliferation capability can be further improved.

The arithmetic average roughness Ra is measured in accordance with a method described in JIS B 0601:2001 (ISO 4287:1997).

In this case, as a specific surface area of the surface of the coating layer 3 becomes larger, antibacterial properties also tend to be improved.

A factor for improvement of osteoconductivity and cell proliferation capability is considered to be that, as the specific surface area of the surface of the coating layer 3 becomes larger, when brought into contact with water (for example, blood), an amount of generation of ammonium ions increases.

Another factor for improvement of antibacterial properties is considered to be that, as the specific surface area of the surface of the coating layer 3 becomes larger, an amount of generation of ammonia increases when brought into contact with water. Antibacterial properties against Gram-positive bacteria particularly tend to be improved.

A maximum height Rz of the base 2 is preferably 0.5 $\mu$m or more and 1000 $\mu$m or less, more preferably 1 $\mu$m or more and 750 $\mu$m or less, and still more preferably 1.5 $\mu$m or more and 500 $\mu$m or less.

On the other hand, a maximum height Rz of the coating layer 3 is preferably 0.5 $\mu$m or more and 1000 $\mu$m or less, more preferably 1 $\mu$m or more and 750 $\mu$m or less, and still more preferably 1.5 $\mu$m or more and 500 $\mu$m or less.

The maximum height Rz is also measured in accordance with a method described in JIS B 0601:2001 (ISO 4287:1997).

A thickness of the coating layer 3 is preferably 50 $\mu$m or less, more preferably 30 $\mu$m or less, still more preferably 15 $\mu$m or less, and particularly preferably 10 $\mu$m or less. The thickness of the coating layer 3 is preferably 0.5 $\mu$m or more, more preferably 0.75 $\mu$m or more, and still more preferably 1 $\mu$m or more. For example, the thickness of the coating layer 3 can be set to 0.5 $\mu$m or more and 30 $\mu$m or less. Such a coating layer 3 can have high mechanical strength while exhibiting sufficient osteoconductivity and antibacterial properties.

When the aerosol deposition method described later is used to form the coating layer 3, the thickness of the coating layer 3 can be adjusted by setting a film-forming time in the aerosol deposition method and a composition, shape, and size of particles P.

An adhesion strength of the coating layer 3 to the base 2 is preferably 15 MPa or more, more preferably 20 MPa or more and 100 MPa or less, and still more preferably 25 MPa or more and 50 MPa or less. A coating layer 3 having such an adhesion strength can be formed relatively easily when an aerosol deposition method described later is used.

The base 2 preferably includes an uneven structure at least in a region to be covered with the coating layer 3. Even with such a base 2, when the aerosol deposition method described later is used, a homogeneous coating layer 3 having a relatively uniform thickness is easily formed.

The biocompatible material 1 described above can be produced, for example, as follows. Hereinafter, a production method of the biocompatible material of the present embodiment is described.

The production method of the biocompatible material of the present embodiment includes a step of preparing the base 2 and particles containing, as a main component, a compound including silicon and nitrogen, and a step of forming, by causing the particles to collide with a surface of the base 2 by an aerosol deposition method so as to adhere thereto, thereby forming a coating layer 3 covering at least a part of the base 2 and obtaining the biocompatible material 1.

The aerosol deposition method can be performed using, for example, the aerosol deposition apparatus shown in FIG. 3. FIG. 3 is a schematic diagram illustrating an example of an aerosol deposition apparatus. An aerosol deposition apparatus 10 shown in FIG. 3 mainly includes an aerosol chamber 20, a film-forming chamber 30, a support mechanism 40, an aerosol supply mechanism 50, a carrier gas supply mechanism 60, and the like.

In the aerosol chamber 20, particles P containing, as a main component, a compound including silicon and nitrogen are accommodated.

An average particle diameter (D50, median diameter) of the particles P is preferably 10 $\mu$m or less, more preferably 0.1 $\mu$m or more and 7.5 $\mu$m or less, and still more preferably 0.5 $\mu$m or more and 5 $\mu$m or less. This makes it possible to eject the particles P from the aerosol supply mechanism 50 at a higher speed. Thus, the coating layer 3 having the above surface roughness (arithmetic average roughness Ra) can be easily formed.

A particle size distribution is measured in accordance with JIS Z 8825:2013 "Particle size analysis-Laser diffraction/scattering methods". In a particle size distribution (cumulative distribution) in which a horizontal axis represents particle size [pm] in a logarithmic scale and a vertical axis represents frequency [vol%], a particle size when a cumulative value from smaller particle sizes reaches 50% of the whole is the average particle diameter (D50).

A BET specific surface area of the particles P may be 0.3 m$^2$/g or more, 0.4 m$^2$/g or more, 0.5 m$^2$/g or more, 2 m$^2$/g or more, 4 m$^2$/g or more, 5 m$^2$/g or more, or 7 m$^2$/g or more. By increasing the BET specific surface area, the coating layer 3 having the above surface roughness (arithmetic average roughness Ra) is likely to be formed.

The BET specific surface area can be adjusted by changing firing conditions (for example, firing temperature and firing time) when synthesizing the compound (producing the particles P) and by changing a size of a raw material powder.

The BET specific surface area of the particles P may be 20 m$^2$/g or less, 15 m$^2$/g or less, 10 m$^2$/g or less, or 6 m$^2$/g or less.

A range of the BET specific surface area may be 0.3 m$^2$/g or more and 20 m$^2$/g or less. The BET specific surface area of the particles P can be measured in accordance with JIS Z 8830:2013 "Methods for determination of specific surface area of powders (solids) by gas adsorption" by a BET one-point method using nitrogen gas.

The particles P containing, as a main component, the composite compound can be produced, for example, by the following procedure.

First, as raw material powders, a silicon nitride powder, a yttria powder, and a silica powder are prepared.

Next, at least two kinds among these are compounded to obtain a mixed powder so as to have a target crystal composition. The raw material powders may be mixed using, for example, a ball mill. The raw material powders may be mixed by wet mixing using a solvent.

Thereafter, after sieving the mixed powder to remove coarse particles, the powder is fired at a temperature of 1500°C or more and 1600°C or less in a nitrogen atmosphere. A firing time may be, for example, 2 hours or more and 20 hours or less.

Subsequently, by performing a disintegration treatment on the resultant fired product using a ball mill or the like, the particles P can be obtained. A crystal phase contained in the particles P can be analyzed by X-ray diffraction.

On the other hand, the particles P containing, as a main component, silicon nitride can be produced by performing surface treatment and washing with water as necessary on a silicon nitride powder obtained by a known method such as a direct nitriding method, an imide thermal decomposition method, or a combustion synthesis method.

A heating section 201 for heating the particles P is provided on an outer periphery of the aerosol chamber 20.

Although a heating temperature of the aerosol chamber 20 is not particularly limited, the temperature is preferably 100°C or more and 300°C or less, and more preferably 150°C or more and 250°C or less.

The support mechanism 40 is provided in the film-forming chamber 30 and is configured to support a film-forming target (the base 2 in this embodiment). The support mechanism 40 includes a fixing portion 401 to fix the base 2 and a drive portion (not shown) to rotate the fixing portion 401 around a central axis and to tilt the fixing portion 401. With such a configuration, a uniform and homogeneous coating layer 3 is likely to be formed on the surface of the base 2.

The aerosol supply mechanism 50 connects the aerosol chamber 20 and the film-forming chamber 30 and has a nozzle 501 that ejects the particles P. The aerosol supply mechanism 50 also has a moving mechanism (not shown) to move the nozzle 501 in an in-plane direction of the base 2. With such a configuration, a uniform and homogeneous coating layer 3 is likely to be formed on the surface of the base 2.

An exhaust device (depressurizing device) 70 is connected to the film-forming chamber 30.

A degree of depressurization of the aerosol chamber 20 and the film-forming chamber 30 is preferably 10 kPa or more and 100 kPa or less, and more preferably 20 kPa or more and 70 kPa or less.

A moving speed of the nozzle 501 (base 2) is preferably about 1 mm/s or more and 30 mm/s or less, and more preferably about 5 mm/s or more and 20 mm/s or less. By moving the nozzle 501 at such a moving speed, uniformity and homogeneity of the coating layer 3 are likely to be enhanced.

A separation distance between the nozzle 501 and the base 2 is preferably 5 mm or more and 30 mm or less, and more preferably 10 mm or more and 20 mm or less.

An angle formed by a normal NL at a position where the particles P collide with the base 2 and a central axis O of the nozzle 501 is preferably 10° or more and 50° or less, and more preferably 20° or more and 40° or less.

In the aerosol deposition apparatus 10, a carrier gas is supplied from the carrier gas supply mechanism 60 to the aerosol chamber 20. Thus, the particles P are dispersed in the carrier gas supplied to the aerosol chamber 20 to generate an aerosol, which is ejected from the nozzle 501 of the aerosol supply mechanism 50 toward the base 2. An ejection speed of the particles P is preferably set to 1 L/min or more and 25 L/min or less, and more preferably set to 5 L/min or more and 15 L/min or less.

A flow velocity and a flow rate of the carrier gas may be appropriately set depending on an ejection amount of the aerosol, a constituent material and an average particle diameter of the particles P ejected from the nozzle 501.

The carrier gas may be supplied to the aerosol chamber 20 as two separated systems, i.e., an original carrier gas to carry the particles P and an lifting gas to blow up the particles P.

As the carrier gas, for example, $O_2$, $N_2$, Ar, He, or air can be used.

When the biocompatible material 1 is produced using the aerosol deposition apparatus 10, first, the base 2 is fixed to the fixing portion 401 of the support mechanism 40.

Thereafter, the aerosol chamber 20 and the film-forming chamber 30 are depressurized. Thus, convection in the film-forming chamber 30 is suppressed, and collision of the particles P with the surface of the base 2 is facilitated.

In this state, by operating the drive portion, rotation and/or tilting of the base 2 is started.

Next, a carrier gas is supplied from the carrier gas supply mechanism 60 to the aerosol chamber 20. Thus, the particles P are dispersed in and accelerated by the carrier gas to generate an aerosol.

Thereafter, the aerosol (particles P) is ejected from the nozzle 501 of the aerosol supply mechanism 50 toward the surface of the base 2. At this time, the nozzle 501 may be moved in an in-plane direction as necessary.

The particles P, by colliding with the base 2, are crushed (ground) and are deposited on the surface of the base 2.

Through the steps above, the coating layer 3 having a predetermined thickness is formed. The steps above may be repeated a plurality of times.

According to the aerosol deposition method described above, the coating layer 3 that covers the surface of the base 2 can be formed while preventing or suppressing alteration (for example, oxidation or compositional change) and deterioration of the compound contained in the particles P. Therefore, the biocompatible material 1 can sufficiently exhibit osteoconductivity and antibacterial properties derived from characteristics of the compound above.

The biocompatible material 1 described above has excellent osteoconductivity and is therefore used for bone or teeth, and is particularly preferably used for bone. By filling, embedding, screwing, or piercing the biocompatible material 1 into bone, fusion with the bone can be caused at an early stage.

As the base 2 having the uneven structure described above, for example, medical devices such as a pin, a bolt, a nut, and a screw can be exemplified. Even on a surface having such an uneven structure (deformed surface), according to the aerosol deposition method, a uniform coating layer 3 can be formed.

Further, the following aspects may also be provided.

(1) A method for producing a biocompatible material, comprising: preparing a base and particles containing, as a main component, a compound including silicon and nitrogen; and causing the particles to collide with a surface of the base by an aerosol deposition method so as to adhere thereto, thereby forming a coating layer covering at least a part of the base and obtaining the biocompatible material.

(2) The method for producing a biocompatible material according to (1), wherein the compound further contains yttrium and oxygen.

(3) The method for producing a biocompatible material according to (2), wherein the compound contains at least one selected from the group consisting of $Si_3N_4$-$SiO_2$-$Y_2O_3$-based compounds and $Si_3N_4$-$Y_2O_3$-based compounds.

(4) The method for producing a biocompatible material according to (2) or (3), wherein the compound contains a composition within a triangular region defined by three points of $Y_5Si_3O_{12}N$, $Y_2Si_3O_3N_4$, and $Y_4Si_2O_7N_2$ in a phase diagram of a ternary system of $Si_3N_4$, $SiO_2$, and $Y_2O_3$.

(5) The method for producing a biocompatible material according to any one of (2) to (4), wherein the compound contains at least one selected from the group consisting of $Y_5Si_3O_{12}N$, $YSiO_2N$, $Y_4Si_2O_7N_2$, and $Y_2Si_3O_3N_4$.

(6) The method for producing a biocompatible material according to (1), wherein the compound contains silicon nitride, and a content of fluorine is 900 mass ppm or less.

(7) The method for producing a biocompatible material according to any one of (1) to (6), wherein when an arithmetic average roughness Ra of a surface of the base is defined as RaB and an arithmetic average roughness Ra of a surface of the coating layer is defined as RaC, RaC/RaB is 0.9 or more and 1.5 or less.

(8) The method for producing a biocompatible material according to any one of (1) to (7), wherein an arithmetic average roughness Ra of the surface of the coating layer is 0.01 $\mu$m or more and 300 $\mu$m or less.

(9) The method for producing a biocompatible material according to any one of (1) to (8), wherein a thickness of the coating layer is 50 $\mu$m or less.

(10) The method for producing a biocompatible material according to any one of (1) to (9), wherein an average particle diameter of the particles is 10 $\mu$m or less.

(11) The method for producing a biocompatible material according to any one of (1) to (10), wherein a BET specific surface area of the particles is 0.3 $m^2$/g or more.

(12) A biocompatible material, comprising: a base; and a coating layer covering at least a part of the base and containing, as a main component, a compound including silicon and nitrogen.

(13) The biocompatible material according to (12), wherein an adhesion strength of the coating layer to the base is 15 MPa or more.

(14) The biocompatible material according to (12) or (13), wherein the base includes an uneven structure in at least a region covered with the coating layer.

(15) The biocompatible material according to any one of (12) to (14), wherein the biocompatible material is used for bone. The present disclosure is not limited thereto.

Finally, various embodiments according to the present disclosure have been described above by way of example, and are not intended to limit a scope of the invention. The novel embodiments can also be implemented in various other forms, and various omissions, replacements, and modifications can be made without departing from the gist of the invention. Such embodiments and their modifications are included in the scope and spirit of the invention and are also encompassed within the scope of the invention set forth in the claims and equivalents thereof.

The biocompatible material described above is not limited to a component (stem) of an artificial joint, and can be used for medical devices such as bone fillers (artificial bone or bone substitute) such as intervertebral spacers and lamina spacers, puncture needles, indwelling needles, stents, scissors, clips, scalpels, forceps, screws, bolts, and screws. The biocompatible material may be a member to be filled into a bone defect portion.

**EXAMPLES**

The present invention is described in greater detail below using the following Examples and Comparative Examples; however, the present invention is not limited to the Examples below.

11. Production of Particles

11-1. Silicon Nitride Particles

A raw material was prepared by compounding silicon powder and calcium fluoride. Calcium fluoride was compounded at a ratio of 1.5 mass% relative to the silicon powder. Using this raw material, a molded body (bulk density: 1.4 g/cm$^3$) was produced and fired at 1400°C for 60 hours in an electric furnace to produce a silicon nitride ingot. The firing atmosphere was a mixed atmosphere of nitrogen and hydrogen ($N_2$:$H_2$ = 80:20, volumetric basis). The obtained ingot was roughly crushed and then wet-milled by a ball mill.

The silicon nitride powder obtained by wet milling underwent a posttreatment by immersion for 2 hours in hydrofluoric acid at 60°C (concentration: 10 mass%). Thereafter, the silicon nitride powder was taken out from hydrofluoric acid, washed with water, and dried in a nitrogen atmosphere. In this manner, silicon nitride particles were obtained.

<Fluorine and Chlorine Contents>

Contents of fluorine and chlorine contained in the silicon nitride particles were measured by the following procedure.

First, the silicon nitride powder was heated using an automatic sample combustion apparatus (manufactured by Mitsubishi Chemical Corporation; model: AQF-2100H), and generated gases were dissolved in water. Next, in accordance with JIS R 1603:2007, fluorine and chlorine dissolved in water were measured using an ion chromatograph (manufactured by Thermo Fisher Scientific; model: ICS-2100). Based on the measured values, the contents of fluorine and chlorine in the silicon nitride particles were 36 mass ppm and 680 mass ppm, respectively.

<Contents of Fe, Al, and Ca>

Using a fluorescent X-ray analyzer (manufactured by Rigaku Corporation; model: ZSX-Primus II), contents of Fe (iron), Al (aluminum), and Ca (calcium) in the silicon nitride particles were measured. The contents were 380 mass ppm, 160 mass ppm, and 1000 mass ppm, respectively.

<Oxygen Content>

An oxygen-nitrogen analyzer (manufactured by HORIBA, Ltd.; model: EMGA-920) was loaded with 0.01 g of silicon nitride particles and 0.01 g of carbon powder. Under a helium gas atmosphere, the temperature was raised from 20°C to 2400°C at a heating rate of 8°C/s. Oxygen and nitrogen released with temperature elevation were detected. From peak areas attributable to desorption of surface oxygen and internal oxygen, the amounts of surface oxygen and internal oxygen were determined. The oxygen content calculated from the sum of these values was 0.94 mass%.

<α-Phase Ratio>

The α-phase ratio of silicon nitride was measured as follows.

Namely, X-ray diffraction of the silicon nitride was performed with CuKα radiation using an X-ray diffractometer (manufactured by Rigaku; model: Ultima IV). The α-phase was represented by diffraction line intensities $I_{a102}$ of the (102) plane and $I_{a210}$ of the (210) plane, and the β-phase was represented by diffraction line intensities $I_{b101}$ of the (101) plane and $I_{b210}$ of the (210) plane. Using these diffraction line intensities, the α-phase ratio calculated by the following equation was 91.0%:

$$\alpha\text{-phase ratio } (\%) = \{(I_{a102} + I_{a210}) \ / \ (I_{a102} + I_{a210} + I_{b101} + I_{b210})\} \times 100$$

<BET Specific Surface Area>

In accordance with JIS Z 8830:2013 "Methods for determination of specific surface area of powders (solids) by gas adsorption," the BET specific surface area of the silicon nitride particles measured by a BET one-point method using nitrogen gas was 8.04 $m^2$/g.

<Particle Size Distribution>

The particle size distribution of silicon nitride particles was measured by a laser diffraction/scattering method.
The measurement was conducted in accordance with the method described in JIS Z 8825:2013 "Particle size analysis-Laser diffraction/scattering method."
In the particle size distribution (cumulative distribution) shown with particle size [$\mu$m] on the horizontal axis in logarithmic scale and frequency [vol%] on the vertical axis, the particle diameter D50 at which the cumulative value from the small particle size reached 50% of the total was 0.72 $\mu$m.

11-2. N-Apatite ($Y_5Si_3O_{12}N$) Particles

First, as raw material powders, 0.7 g of silicon nitride powder (manufactured by UBE Corporation; product name: E10), 11.5 g of yttria powder (manufactured by Shin-Etsu Chemical Co., Ltd.; product name: UUHP), and 2.8 g of silica powder (manufactured by Kojundo Chemical Laboratory Co., Ltd.) were weighed.
The weighed raw material powders, 350 g of silicon nitride balls with a diameter of 16 mm, and 150 mL of ethanol were placed in a container (Nalgene plastic bottle, capacity: 500 mL). The container sealed with a lid was placed on a tabletop ball mill (manufactured by Eishin Co., Ltd.; product name: BM-15), the rotational speed was set to 82 rpm, and premixing was performed for 1 hour under this condition to obtain a mixed slurry.
Subsequently, only the powder was collected from the obtained mixed slurry using a membrane filter. The collected particles were dried at 120°C for 3 hours.
Thereafter, the dried particles were sieved using a nylon sieve (#100).
Next, the particles that passed through the sieve were packed into a forming container made of boron nitride. This forming container was fired at 1550°C for 6 hours under conditions of a nitrogen atmosphere and 0.85 MPa (gauge pressure) using an electric furnace (manufactured by Fuji Dempa Kogyo Co., Ltd.; trade name: Hi-Multi 500).
Next, the particles obtained by firing were placed in a container (Nalgene plastic bottle, capacity: 500 mL).
Then, 375 g of silicon nitride balls having a diameter of 5 mm and 150 mL of ethanol were added to this container, and the container was sealed with a lid. The sealed container was placed on the above-described tabletop ball mill, the rotational speed was set to 82 rpm, and disintegration treatment was performed for 30 hours under this condition.
Thereafter, particles were collected from the slurry after the disintegration treatment using a membrane filter. The collected particles were dried at 110°C for 3 hours.
Next, the dried particles were sieved using a nylon sieve (#330). The particles that passed through the sieve were recovered as particles of an $Si_3N_4$-$SiO_2$-$Y_2O_3$-based compound.
Then, the composition of the particles was examined using an X-ray diffractometer (manufactured by Rigaku Corporation; model: Ultima IV).
As a result, it was confirmed that the particles contained N-apatite ($Y_5Si_3O_{12}N$) as a main component. On the other hand, no components other than N-apatite were detected. The average particle diameter D50 was 1.2 $\mu$m, and the BET specific surface area was 4.36 $m^2$/g.

11-3. N-Wollastonite ($YSiO_2N$) Particles

First, as raw material powders, 3.2 g of silicon nitride powder (manufactured by Ube Corporation; product name: E10), 10.4 g of yttria powder (manufactured by Shin-Etsu Chemical Co., Ltd.; trade name: UUHP), and 1.4 g of silica powder (manufactured by Kojundo Chemical Laboratory Co., Ltd.) were each weighed. Except that these raw material powders were used, the same procedure as that for the N-apatite particles was carried out to obtain particles of an $Si_3N_4$-$SiO_2$-$Y_2O_3$-based compound.
Then, the composition of the particles was examined in the same manner as described above. As a result, the particles were confirmed to contain N-wollastonite ($YSiO_2N$) as a main component. On the other hand, no components other than N-wollastonite were detected.
The average particle diameter D50 was 1.2 $\mu$m, and the BET specific surface area was 4.26 $m^2$/g.

11-4. N-YAM ($Y_4Si_2O_7N_2$) Particles

First, as raw material powders, 1.9 g of silicon nitride powder (manufactured by Ube Corporation; product name: E10), 12.3 g of yttria powder (manufactured by Shin-Etsu Chemical Co., Ltd.; trade name: UUHP), and 0.8 g of silica powder (manufactured by Kojundo Chemical Laboratory Co., Ltd.) were each weighed. Except that these raw material powders were used, the same procedure as that for the N-apatite particles was carried out to obtain particles of an

$Si_3N_4$-$SiO_2$-$Y_2O_3$-based compound.

Then, the composition of the particles was examined in the same manner as described above. As a result, it was confirmed that the particles contained N-YAM ($Y_4Si_2O_7N_2$) as a main component. On the other hand, no components other than N-YAM were detected.

The average particle diameter D50 was 23.83 $\mu$m, and the BET specific surface area was 0.34 $m^2$/g.

11-5. N-Melilite ($Y_2Si_3O_3N_4$) Particles

First, as raw material powders, 5.7 g of silicon nitride powder (manufactured by Ube Corporation; product name: E10) and 9.3 g of yttria powder (manufactured by Shin-Etsu Chemical Co., Ltd.; trade name: UUHP) were each weighed. Except that these raw material powders were used, the same procedure as that for the N-apatite particles was carried out to obtain particles of an $Si_3N_4$-$Y_2O_3$-based compound.

Then, the composition of the powder was examined in the same manner as described above. As a result, it was confirmed that the particles contained N-melilite ($Y_2Si_3O_3N_4$) as a main component. On the other hand, no components other than N-melilite were detected.

The average particle diameter D50 was 17.74 $\mu$m, and the BET specific surface area was 0.49 $m^2$/g.

12. Fabrication of Samples (Biocompatible Materials)

(Example 11)

First, a disk-shaped base A made of a titanium alloy having a diameter of 20 mm and a thickness of 2 mm, and a rectangular base B made of titanium having a width of 10 mm and a length of 40 mm were prepared.

Using the aerosol deposition apparatus 10 shown in FIG. 3, a coating layer was formed on the surfaces of the base A and the base B with silicon nitride particles. In the aerosol deposition apparatus 10, a carrier gas and a lifting gas were supplied in two separated lines. The nozzle was fixed, and an X-Y stage (support mechanism 40) supporting the bases A and B was configured to be movable in an in-plane direction.

The film-forming conditions were as follows:

- Degree of depressurization of the aerosol chamber 20 and the film-forming chamber 30: 31 kPa
- Type of carrier gas and lifting gas: $N_2$
- Flow rate of lifting gas: 5 L/min
- Flow rate of carrier gas: 8 L/min
- Heating temperature of the aerosol chamber 20: 150°C
- Number of laminations: 20 times
- Moving speed of the base: 5 mm/s
- Angle formed by a normal to the surface of the base and the nozzle: 30°
- Separation distance between the nozzle and the surface of the base: 10 mm

Thus, a coating layer having a thickness of 4 $\mu$m was formed on each of the surfaces of the base A and the base B.

(Example 12)

Except that N-apatite ($Y_5Si_3O_{12}N$) particles were used, in the same manner as in Example 11, a coating layer having a thickness of 4 $\mu$m was formed on each of the surfaces of the base A and the base B.

13. Measurement and Evaluation

13-1. Surface Roughness (Arithmetic Average Roughness Ra, Maximum Height Rz)

The surface roughness (arithmetic average roughness Ra and maximum height Rz) of the surface of the base A, and that of the surface of the coating layer in Example 11, was measured in accordance with the method described in JIS B 0601:2001.

As a result, the arithmetic average roughness Ra and the maximum height Rz of the surface of the base A were 0.25 $\mu$m and 1.53 $\mu$m, respectively, and the arithmetic average roughness Ra and the maximum height Rz of the surface of the coating layer in Example 11 were 0.33 $\mu$m and 1.86 $\mu$m, respectively.

13-2. XRD Measurement

XRD measurement was performed on the surface of the base A and the surface of the coating layer in Example 11 using an X-ray diffractometer (manufactured by Rigaku Corporation; model: Ultima IV).

The results are shown in FIG. 4.

As shown in FIG. 4, it was confirmed that silicon nitride was present in the coating layer without alteration.

13-3. Adhesion Test of Coating Layer

A cup made of an epoxy-based thermosetting resin (glass transition temperature: 200°C, coefficient of linear expansion ($\alpha_1$): 15 ppm/°C, coefficient of linear expansion ($\alpha_2$): 55 ppm/°C, hardness at heat: 85, flexural modulus: 13 GPa, flexural strength: 100 MPa) was formed, with the shape and size shown in FIG. 5, on the surface of the base B, and on the surfaces of the coating layers in Example 11 and in Example 12. The units of the numerical values in FIG. 5 are "mm." The resin molding conditions were as follows:

- Mold clamping pressure: 14 t
- Molding pressure: 9 MPa
- Molding time: 120 s
- Molding temperature: 175°C
- Post cure: 175°C $\times$ 6 h

Next, a peeling operation of the cup was performed using a bond tester (manufactured by Nordson Corporation; model: Bondtester 4000). The conditions for this operation were as follows:

- Shear temperature: room temperature
- Speed: 10 $\mu$m/s
- Test height: 100 $\mu$m
- Lower limit of measurement: 0.5 kgf
- Shear direction: short-side direction of the base

The results are shown in FIG. 6.

Peeling occurred at the interface between the cup and the coating layer, and was not observed at the interface between the base B and the coating layer. It is considered that the adhesion strength between the base B and the coating layer is greater than the measured values shown in FIG. 6.

13-4. Antibacterial Test in Sample

For the sample of Example 1, antibacterial properties were evaluated in accordance with the method described in JIS Z 2801:2010 "Antibacterial products-Test for antibacterial activity and efficacy by film covering method."

Specifically, first, 0.1 mL of a test bacterial solution was dropped onto the sample placed in a petri dish, a film (1 cm square) was placed thereon, and the petri dish was covered with a lid.

Next, the petri dish was placed under conditions of 35°C and 90% RH or higher, and cultured for 24 hours.

After 24 hours, 10 mL of SCDLP medium was added to wash out test bacteria from the film and the sample.

Then, the number of test bacteria in the washing solution was measured by an agar plate culture method.

The results are shown in FIG. 7.

As shown in FIG. 7, the sample of Example 11 exhibited high antibacterial properties against both Staphylococcus aureus and Escherichia coli.

In addition, as confirmed by the XRD measurement, silicon nitride was present in the coating layer without alteration. That is, the above excellent antibacterial properties are effects attributable to silicon nitride itself.

Therefore, in the following, antibacterial properties, cell proliferation capability, and osteoconductivity were evaluated for N-apatite ($Y_5Si_3O_{12}N$) particles, N-wollastonite ($YSiO_2N$) particles, N-YAM ($Y_4Si_2O_7N_2$) particles, and N-melilite ($Y_2Si_3O_3N_4$) particles in addition to silicon nitride particles.

14-1. Antibacterial Test in Particles

First, bacteria (Staphylococcus aureus) were pre-cultured in a BHI liquid medium to prepare a bacterial suspension ($1\times10^5$ to $1\times10^6$ CFU/mL). Next, 0.15 g of each particle and 1 mL of distilled water were placed in a microtube to obtain a 15 w/v% dispersion.

Then, after sterilization with ultraviolet light, 1 mL of the above bacterial suspension was added to this microtube. Thereafter, under room temperature (about 20°C), the mixture was stirred with a tube rotator for 5 minutes, the supernatant was collected, and bacterial viability was measured by a WST-8 assay and fluorescence imaging (Live & Dead staining: CFDA, PI, DAPI).

In addition, viability was also measured in the same manner when Staphylococcus epidermidis was used as the bacterium. The results are shown in Table 1. Table 1 also shows, in the column "ref.," the results of viability in the case where no particles were used.

14-2. Cell Proliferation Capability and Osteoconductivity (Calcification) Test in Particles

First, the particles were pulverized with a pestle and mortar, sieved, and adjusted to 75 $\mu$m or less in diameter. Then, using ultrapure water, suspensions of the particles at concentrations of 1 mg/mL and 10 mg/mL were prepared.

Next, the suspensions were dropped onto tissue culture microplates [Iwaki 3820-024 (24-well) or MatTek P06G-0-20-F/H (6-well)] so as to be 0.1 mg/well and 1 mg/well.

After dropping, the microplates were dried in a dry heat sterilizer (TABAI LC-122) at 50°C for 24 hours and sterilized with ultraviolet light. Thus, beds for cell culture were prepared. On the beds prepared in this manner, a proliferation medium and an osteoconductive medium were prepared. The preparation methods of each medium were as follows.

As the proliferation medium, DMEM (Nacalai Tesque) containing 4.5 g/L of glucose supplemented with 10% fetal bovine serum and 1% PS was used. As the osteoconductive medium, the proliferation medium supplemented with 10 mM $\beta$-glycerophosphate, 50 $\mu$g/mL of (L+) ascorbic acid, and 100 nM dexamethasone was used.

A mouse bone marrow-derived KUSA-A1 cell line (JCRB) was selected as the primary choice. The KUSA-A1 cell line was seeded at $5\times10^4$ cells/cm$^2$ in each of the proliferation medium and the osteoconductive medium, and cell proliferation capability and osteoconductivity were evaluated after a predetermined number of days had elapsed.

For the evaluation of cell proliferation capability, WST-8 (manufactured by FUJIFILM Wako Pure Chemical Corporation; product name: Dojindo 341-07761) was used. The measurement method of WST-8 followed the instruction manual attached to the product. Absorbance of the medium after 14 days was measured (spectrophotometer U-3900, manufactured by Hitachi High-Tech Corporation), and the cell proliferation capability was quantified. The results are shown in Table 1. Greater absorbance indicates superior cell proliferation capability. Table 1 also shows, in the column "ref.," the absorbance results in the case where no particles were used.

Osteoconductivity (calcification) was evaluated by estimation using alizarin red S staining on the medium after 7 days. Raman spectra of the stained medium were measured, and the degree of staining was evaluated according to the following criteria:

A: The proportion of the stained portion is larger than in the case where no particles are used.
B: The proportion of the stained portion is equivalent to that in the case where no particles are used.
C: The proportion of the stained portion is smaller than in the case where no particles are used.

These results are also shown in Table 1.

[Table 1]

TABLE 1

| ITEM | | ref. | SILICON NITRIDE | N-APATITE | N-WOLLASTONITE | N-YAM | N-MERILLITE |
|---|---|---|---|---|---|---|---|
| D50 [μm] | | - | 0.72 | 1.20 | 1.20 | 23.83 | 17.74 |
| BET SPECIFIC SURFACE AREA [m²/g] | | - | 8.04 | 4.36 | 4.26 | 0.34 | 0.49 |
| ANTI BACTERIAL PROPERTY | STAPHYLOCOCCUS AUREUS | 0.72 | 0.02 | 0.38 | 0.02 | 0.74 | 0.75 |
| | STAPHYLOCOCCUS EPIDERMIDIS | 2.00 | 0.05 | 0.10 | 0.05 | 1.77 | 1.86 |
| OSTEOCONDUCTIVITY | | - | B | A | A | B | B |
| CELL PROLIFERATION CAPABILITY | | 0.81 | 1.11 | 1.42 | 1.40 | 1.52 | 1.48 |

As shown in Table 1, each particle had sufficient osteoconductivity and antibacterial properties. Accordingly, in the case where a biocompatible material is produced by forming, by aerosol deposition, a coating layer composed mainly of a compound containing silicon and nitrogen so as to cover a base, sufficient osteoconductivity and antibacterial properties are considered to be exhibited even in a living body into which the material is transplanted. Therefore, safe treatment can be performed for a patient. In addition, even when the type of base is changed or the thickness of the coating layer is changed, the same effects as described above are considered to be obtained.

21. Preparation of Base

Disk-shaped bases C and D, each having a diameter of 14 mm and a thickness of 2 mm, were prepared by molding a titanium alloy powder.

22. Fabrication of Samples (Biocompatible Materials)

(Example 21)

Except that the angle formed by a normal to the surface of the base and the nozzle was set to 0°, the same procedure as in Example 11 was performed to form a coating layer on the surface of the base C.

(Example 22)

Except that the angle formed by a normal to the surface of the base and the nozzle was set to 15°, the same procedure as in Example 11 was performed to form a coating layer on the surface of the base D.

23. Measurement and Evaluation

23-1. Surface Roughness (Arithmetic Average Roughness Ra, Maximum Height Rz)

The surface roughness (arithmetic average roughness Ra and maximum height Rz) of the base C, the surface of the coating layer in Example 21, the base D, and the surface of the coating layer in Example 22 were measured in the same manner as in "13-1. Surface Roughness."
As a result, the arithmetic average roughness Ra and the maximum height Rz of the surface of the base C were 150 $\mu$m and 778 $\mu$m, respectively, and those of the surface of the coating layer in Example 21 were 149 $\mu$m and 752 $\mu$m, respectively.
On the other hand, the arithmetic average roughness Ra and the maximum height Rz of the surface of the base D were 20.2 $\mu$m and 109 $\mu$m, respectively, and those of the surface of the coating layer in Example 22 were 20.6 $\mu$m and 122 $\mu$m, respectively.

23-2. Surface SEM Observation and Composition Analysis

The surfaces of the samples of Example 21 and Example 22 were observed at an accelerating voltage of 15.0 kV, a working distance (WD) of about 10 mm, and a magnification of 10000×, using a field emission scanning electron microscope (FE-SEM) (manufactured by JEOL Ltd.; model: JSM-7001F/SHL). EDX analysis was performed using an EDX analyzer (manufactured by JEOL Ltd.; "JED-2300")
The results are shown in FIG. 8 (Example 21) and FIG. 9 (Example 22).
FIG. 8 is a view showing results of surface SEM observation and composition analysis in the sample of Example 21. FIG. 9 is a view showing results of surface SEM observation and composition analysis in the sample of Example 22.
As shown in FIG. 8, in the sample of Example 21, components (Si and N) derived from film-forming particles were strongly detected at convex portions, but the detection sensitivity of components derived from film-forming particles decreased at concave portions. On the other hand, as shown in FIG. 9, in the sample of Example 22, in addition to components derived from film-forming particles, components (Ti and Al) derived from the base were detected.

23-3. Cross-sectional SEM Observation and Composition Analysis

The cross sections of the samples of Example 21 and Example 22 were observed at an accelerating voltage of 15.0 kV, a working distance (WD) of about 10 mm, and a magnification of 10000×, using a field emission scanning electron microscope (FE-SEM) (manufactured by JEOL Ltd.; model: JSM-7001F/SHL). EDX analysis was performed using an EDX analyzer (manufactured by JEOL Ltd.; "JED-2300").

The results are shown in FIG. 10 (Example 21) and FIG. 11 (Example 22).

FIG. 10 is a view showing results of cross-sectional SEM observation and composition analysis in the sample of Example 21. FIG. 11 is a view showing results of cross-sectional SEM observation and composition analysis in the sample of Example 22.

As shown in FIG. 10, in the sample of Example 21, it was confirmed that a homogeneous and dense coating layer having a thickness of about 1 $\mu$m was formed. As shown in FIG. 11, it was also confirmed that, in the sample of Example 22, a homogeneous and dense coating layer having a thickness of about 1 $\mu$m was formed.

31. Preparation of Bases

Ten bases D and ten bases E having a smooth surface (arithmetic average roughness Ra: 0.25 $\mu$m, maximum height Rz: 1.57 $\mu$m) were prepared.

32. Fabrication of Samples (Biocompatible Materials)

(Example 31-1)

On surfaces of five bases D, except that the angle formed by a normal to the surface of the base and the nozzle was set to 15°, the same procedure as in Example 11 was performed to form coating layers.

(Example 31-2)

On surfaces of five bases D, except that the angle formed by a normal to the surface of the base and the nozzle was set to 15°, the same procedure as in Example 12 was performed to form coating layers.

(Example 32-1)

On surfaces of five bases E, coating layers were formed in the same manner as in Example 11.

(Example 32-2)

On surfaces of five bases E, coating layers were formed in the same manner as in Example 12.

33. Measurement

33-1. Adhesion Strength Test of Coating Layer

For the samples of Example 31-1, Example 31-2, Example 32-1, and Example 32-2, adhesion strength tests were performed, respectively.

The adhesion strength test was carried out using the apparatus shown in FIG. 12.

FIG. 12 is a schematic diagram showing a configuration of an adhesion strength test apparatus.

As shown in FIG. 12, in an adhesion strength test apparatus 100, the sample was disposed so that the coating layer 3 was in contact with a support 102. The support 102 was a cylinder having an inner diameter of 4 mm and an outer diameter of 6 mm. A reinforcing plate 101 was placed on the base 2.

A bottom surface 103a of a stud pin 103 was bonded to the coating layer 3 with an adhesive 105. At this time, the adhesive force was 700 kg/cm$^2$ or more. Then, through a gripper 104 fixed to the opposite end of the bottom surface 103a of the stud pin 103, a load was gradually applied downward (in the direction of the arrow in FIG. 12). The load was increased at a rate of 3 kg/s, and an adhesion strength (MPa) was calculated from the load at which the stud pin 103 separated from the sample. In addition, the layer in which peeling occurred was visually confirmed. It is considered that the portion where peeling occurred is the weakest portion in adhesion in the thickness direction of the sample.

In the samples of Examples 31-1 and 31-2, Hardlock C-355-20 (manufactured by Denka Company Limited) was used as the adhesive 105; in the samples of Examples 32-1 and 32-2, a standard epoxy adhesive of the apparatus manufacturer was used as the adhesive 105.

The average adhesion strength measured in the sample of Example 31-1 was 30.3 MPa, and the average adhesion strength measured in the sample of Example 31-2 was 28.4 MPa. In addition, the peeling mode was material failure of the adhesive 105, and it was confirmed that the adhesion strength of the coating layer 3 to the base 2 was 15 MPa or more.

On the other hand, the average adhesion strength measured in the sample of Example 32-1 was 89.1 MPa, and that measured in the sample of Example 32-2 was 87.4 MPa. The peeling mode was material failure of the adhesive 105, and it was confirmed that the adhesion strength of the coating layer 3 to the base 2 was 15 MPa or more.

41. Preparation of Base

Two of the above bases C were prepared.

42. Fabrication of Sample (Biocompatible Material)

(Example 41)

Except that the angle formed by a normal to the surface of the base and the nozzle was set to 0°, the same procedure as in Example 11 was performed to form a coating layer on the surface of the base C.

(Example 42)

Except that the angle formed by a normal to the surface of the base and the nozzle was set to 0°, the same procedure as in Example 12 was performed to form a coating layer on the surface of the base C.

43. Antibacterial Test for Samples

For the samples of Examples 41 and 42, antibacterial properties were evaluated in the same manner as "13-4. Antibacterial Test for Samples."
The results are shown in FIG. 13.
FIG. 13 is a graph showing results of antibacterial tests for the samples of Examples 41 and 42.
The antibacterial activity value (R) is calculated on the basis of $R = (U_1 - U_0) - (At - U_0)$.
Here, $U_0$ is the average logarithmic value of the number of viable bacteria immediately after inoculation of an untreated specimen, $U_1$ is the average logarithmic value of the number of viable bacteria of an untreated specimen after 24 hours, and At is the average logarithmic value of the number of viable bacteria of an antibacterial-treated specimen after 24 hours.
As shown in FIG. 13, the sample of Example 41 exhibited antibacterial properties exceeding a target value (antibacterial activity value: 2 or more).

51. Preparation of Base

A screw (manufactured by MISUMI Group Inc.; "cross-recessed A pan tapping screw") shown in FIG. 14B was prepared.
The material of the screw was stainless steel, and the dimensions were 8 mm in diameter and 100 mm in length.

52. Fabrication of Samples (Biocompatible Materials)

Except that no surface treatment was performed on the surface of the screw, the screw was rotated at 10 rpm about its central axis, the moving speed of the base was set to 1 mm/s, the angle formed between the nozzle and the normal to the surface of the base was set to 0°, and the distance between the nozzle and the surface of the base was set to 15 mm, a coating layer was formed in the same manner as in Example 11. The results are shown in FIG. 14.
FIG. 14 is a photograph showing the appearance of the screw before and after formation of the coating layer.
As shown in FIG. 14(a), in appearance, the coating layer appeared to be uniformly formed on the surface of the screw.

53. Measurement and Evaluation

53-2. Surface SEM Observation and Composition Analysis

The surfaces of a thread root and a thread crest of the coated screw (sample) were observed using a field emission scanning electron microscope (FE-SEM) (manufactured by JEOL Ltd.; "JSM-7001F/SHL") under an accelerating voltage of 15.0 kV, a working distance (WD) of about 10 mm, and a magnification of 10,000 times. EDX analysis was performed using an EDX analyzer (manufactured by JEOL Ltd.; "JED-2300").
The results are shown in FIG. 15 (thread root) and FIG. 16 (thread crest).

FIG. 15 is a view showing results of surface SEM observation and composition analysis at the thread root of the coated screw. FIG. 17 is a view showing results of surface SEM observation and composition analysis at the thread crest of the coated screw.

As shown in FIG. 15, in the thread root of the screw, formation of the coating layer was confirmed. As shown in FIG. 16, the coating layer was confirmed to be formed in the thread crest of the screw as well; however, a slight variation in the degree of coating appeared to be present.

53-3. Cross-Sectional SEM Observation and Composition Analysis

The cross section of the thread root of the coated screw (sample) was observed using a field emission scanning electron microscope (FE-SEM) (manufactured by JEOL Ltd.; "JSM-7001F/SHL") under an accelerating voltage of 15.0 kV, a working distance (WD) of about 10 mm, and a magnification of 10,000 times. EDX analysis was performed using an EDX analyzer (manufactured by JEOL Ltd.; "JED-2300"). The results are shown in FIG. 17.

FIG. 17 is a view showing results of cross-sectional SEM observation and composition analysis at the thread root of the coated screw.

As shown in FIG. 17, a homogeneous and dense coating layer having a thickness of about 3 $\mu$m to 3.5 $\mu$m was confirmed to be formed.

[Reference Signs List]

1: biocompatible material.
11: insertion portion.
12: mounting portion.
2: base.
3: coating layer.
10: aerosol deposition apparatus.
20: aerosol chamber.
201: heating section.
30: film-forming chamber.
40: support mechanism.
401: fixing portion.
50: aerosol supply mechanism.
501: nozzle.
60: carrier gas supply mechanism.
70: exhaust device.
100: adhesion strength test apparatus.
101: reinforcing plate.
102: support base.
103: stud pin.
103a: bottom surface.
104: gripper.
105: adhesive.
A: base.
B: base.
P: particles.
NL: normal.
O: central axis.

**Claims**

**1.** A method for producing a biocompatible material, comprising:

preparing a base and particles containing, as a main component, a compound including silicon and nitrogen; and
causing the particles to collide with a surface of the base by an aerosol deposition method so as to adhere thereto, thereby forming a coating layer covering at least a part of the base and obtaining the biocompatible material.

2. The method for producing a biocompatible material according to claim 1, wherein the compound further contains yttrium and oxygen.

3. The method for producing a biocompatible material according to claim 2, wherein the compound contains at least one selected from the group consisting of $Si_3N_4$-$SiO_2$-$Y_2O_3$-based compounds and $Si_3N_4$-$Y_2O_3$-based compounds.

4. The method for producing a biocompatible material according to claim 2 or 3, wherein the compound contains a composition within a triangular region defined by three points of $Y_5Si_3O_{12}N$, $Y_2Si_3O_3N_4$, and $Y_4Si_2O_7N_2$ in a phase diagram of a ternary system of $Si_3N_4$, $SiO_2$, and $Y_2O_3$.

5. The method for producing a biocompatible material according to any one of claims 2 to 4, wherein the compound contains at least one selected from the group consisting of $Y_5Si_3O_{12}N$, $YSiO_2N$, $Y_4Si_2O_7N_2$, and $Y_2Si_3O_3N_4$.

6. The method for producing a biocompatible material according to claim 1, wherein the compound contains silicon nitride, and a content of fluorine is 900 mass ppm or less.

7. The method for producing a biocompatible material according to any one of claims 1 to 6, wherein when an arithmetic average roughness Ra of a surface of the base is defined as RaB and an arithmetic average roughness Ra of a surface of the coating layer is defined as RaC, RaC/RaB is 0.9 or more and 1.5 or less.

8. The method for producing a biocompatible material according to any one of claims 1 to 7, wherein an arithmetic average roughness Ra of the surface of the coating layer is 0.01 $\mu$m or more and 300 $\mu$m or less.

9. The method for producing a biocompatible material according to any one of claims 1 to 8, wherein a thickness of the coating layer is 50 $\mu$m or less.

10. The method for producing a biocompatible material according to any one of claims 1 to 9, wherein an average particle diameter of the particles is 10 $\mu$m or less.

11. The method for producing a biocompatible material according to any one of claims 1 to 10, wherein a BET specific surface area of the particles is 0.3 $m^2$/g or more.

12. A biocompatible material, comprising:

   a base; and
   a coating layer covering at least a part of the base and containing, as a main component, a compound including silicon and nitrogen.

13. The biocompatible material according to claim 12, wherein an adhesion strength of the coating layer to the base is 15 MPa or more.

14. The biocompatible material according to claim 12 or 13, wherein the base includes an uneven structure in at least a region covered with the coating layer.

15. The biocompatible material according to any one of claims 12 to 14, wherein the biocompatible material is used for bone.

FIG. 1

FIG. 2

FIG. 3

FIG. 4

40

5 | 10 | 10 | 10

10

2.9

4

$\phi$ 3.57 (ADHESION SURFACE)

FIG. 5

FIG. 6

FIG. 7

(Example 21)

MASS %

| | C | N | O | Al | Si | Ti | V | TOTAL |
|---|---|---|---|---|---|---|---|---|
| 008 | 9.76 | 21.69 | 8.70 | 1.61 | 32.53 | 25.03 | 0.67 | 100.00 |
| 009 | 6.17 | 18.46 | 8.30 | 1.63 | 31.57 | 32.63 | 1.24 | 100.00 |

MASS %

| | C | N | O | Al | Si | Ti | V | TOTAL |
|---|---|---|---|---|---|---|---|---|
| 006 | 15.76 | 5.84 | 17.19 | 3.10 | 7.23 | 47.48 | 3.41 | 100.00 |
| 007 | 8.26 | 24.64 | 9.77 | 2.76 | 18.50 | 35.28 | 0.78 | 100.00 |

FIG. 8

(Example 22)

MASS %

| | C | N | O | Al | Si | Ti | V | TOTAL |
|---|---|---|---|---|---|---|---|---|
| 044 | 7.01 | 23.26 | 8.67 | 2.21 | 23.14 | 33.70 | 2.03 | 100.00 |
| 045 | 5.91 | 13.66 | 6.58 | 0.67 | 29.94 | 41.57 | 1.67 | 100.00 |
| 046 | 10.35 | 15.13 | 10.36 | 3.07 | 10.10 | 48.04 | 2.94 | 100.00 |

FIG. 9

(Example 21)

MASS %

|     | C      | N      | O     | Al    | Si     | Ti     | V     | TOTAL |
|-----|--------|--------|-------|-------|--------|--------|-------|-------|
| 001 | 73.85  | 0.00   | 4.72  | 0.20  | 16.41  | 4.29   | 0.54  | 100   |
| 002 | 18.07  | 29.95  | 9.08  | 0.48  | 38.07  | 4.09   | 0.26  | 100   |
| 003 | 1.85   | 0.00   | 0.00  | 5.86  | 0.16   | 88.44  | 3.69  | 100   |

FIG. 10

(Example 22)

FIG. 11

FIG. 12

FIG. 13

FIG. 14

FIG. 15

FIG. 16

(THREAD ROOT)

FIG. 17

# EP 4 751 743 A1

## INTERNATIONAL SEARCH REPORT

International application No.

**PCT/JP2024/026354**

### A. CLASSIFICATION OF SUBJECT MATTER

*A61L 27/42*(2006.01)i; *A61L 27/04*(2006.01)i; *A61L 27/30*(2006.01)i; *A61L 27/54*(2006.01)i; *A61L 27/58*(2006.01)i
FI:  A61L27/42; A61L27/04; A61L27/30; A61L27/54; A61L27/58

According to International Patent Classification (IPC) or to both national classification and IPC

### B. FIELDS SEARCHED

Minimum documentation searched (classification system followed by classification symbols)

A61L27/42; A61L27/04; A61L27/30; A61L27/54; A61L27/58

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2023-18024 A (SINTX TECHNOLOGIES, INC.) 07 February 2023 (2023-02-07) claims 1, 7, 12-13, 18, paragraphs [0004]-[0006], [0012], [0024]-[0031], [0071], [0085]-[0086], fig. 5 | 12-15 |
| Y | | 1-3, 6-11 |
| A | | 4-5 |
| X | JP 2023-523604 A (SINTX TECHNOLOGIES, INC.) 06 June 2023 (2023-06-06) claims 1-2, 11-12, paragraphs [0024], [0030]-[0034], [0039]-[0040], [0049], [0085]-[0089], [0092], [0115], [0134], [0143], fig. 4 | 12-15 |
| Y | | 1-3, 6-11 |
| A | | 4-5 |

☑ Further documents are listed in the continuation of Box C.     ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **11 September 2024** | **24 September 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

36

## INTERNATIONAL SEARCH REPORT

| International application No. |
| --- |
| **PCT/JP2024/026354** |

### C. DOCUMENTS CONSIDERED TO BE RELEVANT

| Category* | Citation of document, with indication, where appropriate, of the relevant passages | Relevant to claim No. |
| --- | --- | --- |
| X | JP 2023-523605 A (SINTX TECHNOLOGIES, INC.) 06 June 2023 (2023-06-06) claims 1, 6-7, 13, 15-17, paragraphs [0021], [0026]-[0030], [0035], [0044], [0079]-[0083], [0086], [0109], fig. 4 | 12-15 |
| Y | | 1-3, 6-11 |
| A | | 4–5 |
| X | WO 2022/216951 A1 (SINTX TECHNOLOGIES, INC.) 13 October 2022 (2022-10-13) claims 1, 5-9, 12-13, 15, 17, page 6, paragraph [0005], pages 6-7, paragraph [0008], pages 8-9, paragraphs [0016]-[0017], pages 14-15, paragraphs [0039]-[0041] | 12-15 |
| Y | | 1-3, 6-11 |
| A | | 4–5 |
| Y | JP 2009-189683 A (UNIV. SAGA) 27 August 2009 (2009-08-27) claim 1, paragraphs [0001], [0018], [0026]-[0027] | 1-3, 6-11 |
| A | | 4-5, 12-15 |
| Y | WO 2019/162943 A1 (YEDA RESEARCH AND DEVELOPMENT CO., LTD.) 29 August 2019 (2019-08-29) claims 1, 15-16, 20, 22-24, paragraphs [0013], [0049] | 1-3, 6-11 |
| A | | 4-5, 12-15 |
| Y | WO 2011/056013 A2 (LEE, Y. C.) 12 May 2011 (2011-05-12) claims 1, 3, 9, 13, 15, page 7, line 25 to page 8 line 11, page 9 lines 12-15, page 11 line 23- page 12, line 10 | 1-3, 6-11 |
| A | | 4-5, 12-15 |

Form PCT/ISA/210 (second sheet) (July 2022)

**INTERNATIONAL SEARCH REPORT**
Information on patent family members

International application No.

**PCT/JP2024/026354**

| Patent document cited in search report | | | Publication date (day/month/year) | Patent family member(s) | | | Publication date (day/month/year) |
|---|---|---|---|---|---|---|---|
| JP | 2023-18024 | A | 07 February 2023 | US | 2021/0008254 | A1 | |
| | | | | claims 1-4, 10-11, 16-17, paragraphs [0005]-[0007], [0025], [0037]-[0044], [0084], [0098]-[0099], fig. 5A, 5B | | | |
| | | | | WO | 2018/182835 | A1 | |
| | | | | EP | 4176905 | A1 | |
| | | | | CA | 3055721 | A1 | |
| | | | | CN | 110461378 | A | |
| | | | | KR | 10-2019-0129041 | A | |
| JP | 2023-523604 | A | 06 June 2023 | US | 2021/0331274 | A1 | |
| | | | | claims 1-2, 11-12, paragraphs [0116], [0122]-[0126], [0131]-[0132], [0141], [0176]-[0180], [0183], [0206], [0226], [0235], fig. 4 | | | |
| | | | | WO | 2021/217089 | A1 | |
| | | | | CN | 115515655 | A | |
| | | | | KR | 10-2023-0008125 | A | |
| JP | 2023-523605 | A | 06 June 2023 | US | 2021/0330860 | A1 | |
| | | | | claims 1, 6-7, 13, 15-17, paragraphs [0104], [0109]-[0113], [0118], [0127], [0162]-[0166], [0169], [0192], fig. 4 | | | |
| | | | | WO | 2021/216872 | A1 | |
| | | | | CN | 115702013 | A | |
| | | | | KR | 10-2023-0008753 | A | |
| | | | | CA | 3175606 | A1 | |
| WO | 2022/216951 | A1 | 13 October 2022 | JP | 2024-513573 | A | |
| | | | | US | 2022/0322676 | A1 | |
| | | | | KR | 10-2023-0165816 | A | |
| | | | | CA | 3214652 | A1 | |
| JP | 2009-189683 | A | 27 August 2009 | (Family: none) | | | |
| WO | 2019/162943 | A1 | 29 August 2019 | US | 2020/0392001 | A1 | |
| | | | | US | 2022/0049087 | A1 | |
| | | | | EP | 4173647 | A1 | |
| WO | 2011/056013 | A2 | 12 May 2011 | KR | 10-2011-0050308 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003286073 A